# EUROPEAN PATENT APPLICATION

(11) **EP 3 770 137 A1**
(43) Date of publication of application: **27.01.2021**
(21) Application number: 20195876.6
(22) Date of filing: 31.07.2017
(51) Int. Cl.: C07C 5/327, C07C 5/48, C07C 11/04

(54) **ETHYLENE PRODUCTION PROCESS AND CHEMICAL COMPLEX**

(30) Priority: 02.08.2016 EP 16182435
(62) Divisional of application: 17743362.0
(71) Applicant: Shell Internationale Research Maatschappij B.V., 2596 HR The Hague (NL)
(72) Inventor: MITKIDIS, Georgios, 1031 HW Amsterdam (NL); VAN ROSSUM, Guus, 1031 HW Amsterdam (NL); SAN ROMAN MACIA, Maria, 3747 Ras Laffan (QA); SHAH, Vatsal, Mukundlal, Houston, Texas 77002-4901 (US)
(74) Representative: Shell Legal Services IP

(57) **Abstract**

The invention relates to a process for the production of ethylene from naphtha and ethane, comprising:

subjecting a stream comprising naphtha to steam cracking conditions in a steam cracker unit which is part of a steam cracker configuration, resulting in a stream comprising unconverted ethane, ethylene and hydrogen;

feeding a stream comprising unconverted ethane, ethylene and hydrogen to a separation unit which is part of the steam cracker configuration, and separating said stream in said separation unit into a stream comprising hydrogen and a stream comprising unconverted ethane and ethylene;

feeding a stream comprising unconverted ethane and ethylene to a C2 separation unit which is part of the steam cracker configuration, and separating said stream in the C2 separation unit into a stream comprising ethylene and a stream comprising unconverted ethane;

subjecting a stream comprising ethane to oxidative dehydrogenation (ODH) conditions in an ODH unit which is part of an ODH configuration, resulting in a stream comprising unconverted ethane, ethylene and water;

feeding a stream comprising unconverted ethane, ethylene and water to a water condensation unit which is part of the ODH configuration, and removing water from said stream by condensation in the water condensation unit, resulting in a stream comprising unconverted ethane and ethylene; and

feeding an effluent coming from the ODH configuration, which effluent comprises unconverted ethane and ethylene, to the steam cracker configuration.

## Description

### Field of the invention

The present invention relates to a process for the production of ethylene from ethane, and to a chemical complex which is suitable for performing such ethylene production process.

### Background of the invention

It is known to produce ethylene from ethane by steam cracking an ethane stream under the influence of heat into a product stream comprising ethylene and hydrogen. Before any subsequent step wherein the ethylene is further converted into useful chemical intermediates, the ethylene containing product stream has to be purified. In addition to ethylene and hydrogen, a steam cracker product stream may also contain acetylene and unconverted ethane. Further, said product stream may contain some carbon monoxide and carbon dioxide as impurities. Carbon dioxide may be produced in the presence of oxygen (resulting from some small air ingress into the steam cracker unit) and/or in water-shift reactions of hydrocarbons. Further, carbon monoxide and carbon dioxide may be present as contaminants in the feed. Still further, said product stream may contain methane and C3+ hydrocarbons which impurities may be formed during steam cracking of ethane which is generally a non-catalytic, non-selective conversion process. The latter impurities may also originate from the ethane feed stream. Components other than ethylene need to be removed from the product stream as they may interfere in any subsequent step wherein ethylene is further converted.

Generally, carbon dioxide is removed from the steam cracker product stream by passing the stream through a carbon dioxide removal unit wherein it may be contacted with an aqueous solution of a base, for example sodium hydroxide (caustic solution wash). Hydrogen and methane may be separated from the other components by cryogenic distillation. Alternatively, hydrogen and methane may be separated together with the C2 hydrocarbons, comprising ethylene, any unconverted ethane and any acetylene, from any C3+ hydrocarbons in the product stream. The C2 hydrocarbons then need to be separated from the resulting stream comprising hydrogen, methane and C2 hydrocarbons. Acetylene may be removed by hydrogenating it into ethylene. Finally, ethane has to be separated from the ethylene, which may also be done by distillation. Having to remove all these components from the ethylene containing steam cracker product stream is very cumbersome and results in a relatively high expenditure for producing ethylene. For example, it is known to separate ethane from ethylene, by means of cryogenic distillation in so-called "C2 splitter" columns. In such cryogenic distillation, a relatively high pressure and a relatively low (cryogenic) temperature are applied to effect the separation of ethane from ethylene.

The downstream section of an ethane steam cracker configuration, which downstream section may comprise multiple units for performing the above-described functions of carbon dioxide removal, separation of components (for example by distillation) and acetylene hydrogenation, has a certain capacity which is determined by that unit which has the lowest capacity. In general, there may be a situation wherein the capacity of a steam cracker unit upstream of the downstream section in the steam cracker configuration does not match the higher capacity of the downstream section. In such case, the capacity of the downstream section is not fully utilized leading to a technically disadvantageous, inefficient and less affordable process for the production of ethylene from ethane.

The above-mentioned situation wherein the capacity of a steam cracker unit (comprising one or more furnaces) upstream of the downstream section in an ethane steam cracker configuration does not match the higher capacity of the downstream section, may generally occur when there is a bottleneck in an existing steam cracker configuration or when there is a wish to expand the total capacity of a steam cracker configuration. This may involve a case where a steam cracker furnace is close to or at the end of its life time. Further, this may involve a case where there is a desire to utilize the potential spare capacity in the back-end separation section of an existing steam cracker configuration because of additional design margin (overdesign of distillation towers or installation of higher capacity internals) and/or lower performance of the steam cracking furnace(s) than designed. The latter may also be a result of revamping an existing liquid steam cracker configuration to a gas (ethane) steam cracker configuration. Still further, this may apply to situations wherein 1 or more of a multitude of steam crackers has/have to be taken out of service (e.g. for maintenance or for any other reason).

An object of the present invention is to provide a process for the production of ethylene from ethane, which process may be a technically advantageous, efficient and affordable process and which process involves steam cracking of ethane into ethylene and hydrogen in a steam cracker unit which is part of a steam cracker configuration, more in particular in a situation wherein the capacity of the steam cracker unit upstream of the downstream section in the steam cracker configuration does not match the higher capacity of the downstream section. Such technically advantageous process would preferably result in a lower energy demand and/or lower capital expenditure.

### Summary of the invention

Surprisingly it was found that the above-mentioned object can be achieved by feeding an effluent from an oxidative dehydrogenation (ODH) configuration to the steam cracker configuration, in which ODH configuration ethane is oxidatively dehydrogenated into ethylene and water, and wherein the effluent coming from the ODH configuration that is fed to the steam cracker configuration comprises unconverted ethane and ethylene.

Accordingly, the present invention relates to a process for the production of ethylene from ethane, comprising:
subjecting a stream comprising ethane to steam cracking conditions in a steam cracker unit which is part of a steam cracker configuration, resulting in a stream comprising unconverted ethane, ethylene and hydrogen;
feeding a stream comprising unconverted ethane, ethylene and hydrogen to a separation unit which is part of the steam cracker configuration, and separating said stream in said separation unit into a stream comprising hydrogen and a stream comprising unconverted ethane and ethylene;
feeding a stream comprising unconverted ethane and ethylene to a C2 separation unit which is part of the steam cracker configuration, and separating said stream in the C2 separation unit into a stream comprising ethylene and a stream comprising unconverted ethane;
optionally recycling unconverted ethane from the stream comprising unconverted ethane coming from the C2 separation unit to the steam cracker unit;
subjecting a stream comprising ethane to oxidative dehydrogenation (ODH) conditions in an ODH unit which is part of an ODH configuration, resulting in a stream comprising unconverted ethane, ethylene and water;
feeding a stream comprising unconverted ethane, ethylene and water to a water condensation unit which is part of the ODH configuration, and removing water from said stream by condensation in the water condensation unit, resulting in a stream comprising unconverted ethane and ethylene;
feeding an effluent coming from the ODH configuration, which effluent comprises unconverted ethane and ethylene, to the steam cracker configuration;
optionally recycling unconverted ethane from the stream comprising unconverted ethane coming from the C2 separation unit to the ODH unit.

Further, the present invention relates to a chemical complex which comprises a steam cracker configuration and an oxidative dehydrogenation (ODH) configuration, wherein:
the steam cracker configuration comprises a steam cracker unit, a carbon dioxide removal unit, an optional drying unit, a 1^{st} separation unit, a 2^{nd} separation unit, an optional acetylene hydrogenation unit and a C₂ separation unit, wherein each unit comprises one or more feed lines and one or more effluent lines;
the ODH configuration comprises an ODH unit, a water condensation unit, an optional carbon dioxide removal unit and an optional drying unit, wherein each unit comprises one or more feed lines and one or more effluent lines;
the ODH configuration is integrated with the steam cracker configuration at a position upstream of the optional acetylene hydrogenation unit and upstream of the C2 separation unit; and
no feed line of the ODH unit is integrated with a feed line or effluent line of a unit of the steam cracker configuration, with the exception that a feed line of the steam cracker unit and a feed line of the ODH unit may be integrated, and/or that an optional recycle effluent line of the C2 separation unit and a feed line of the ODH unit may be integrated.

### Brief description of the drawings

Figure 1 depicts an embodiment of the present invention wherein an effluent from a water condensation unit of the oxidative dehydrogenation (ODH) configuration, said effluent comprising unconverted ethane and ethylene, is fed to the steam cracker configuration.
Figure 2 depicts an embodiment of the present invention wherein an effluent from a carbon dioxide removal unit of the ODH configuration, said effluent comprising unconverted ethane and ethylene, is fed to the steam cracker configuration.
Figure 3 depicts an embodiment of the present invention wherein an effluent from a drying unit of the ODH configuration, said effluent comprising unconverted ethane and ethylene, is fed to the steam cracker configuration.

### Detailed description of the invention

In the integrated process of the present invention, use is made of both a steam cracker configuration and an oxidative dehydrogenation (ODH) configuration.

The above-mentioned steam cracker configuration comprises:
(a) a steam cracker unit wherein a stream comprising ethane is subjected to steam cracking conditions, resulting in a stream comprising unconverted ethane, ethylene and hydrogen;
(b) a separation unit wherein a stream comprising unconverted ethane, ethylene and hydrogen is separated into a stream comprising hydrogen and a stream comprising unconverted ethane and ethylene;
(c) a C2 separation unit wherein a stream comprising unconverted ethane and ethylene is separated into a stream comprising ethylene and a stream comprising unconverted ethane.

Further, in the present invention, the steam cracker configuration may comprise a carbon dioxide removal unit, a drying unit, one or more separation units other than the above-mentioned C2 separation unit, an acetylene hydrogenation unit and/or one or more compressors.

The above-mentioned oxidative dehydrogenation (ODH) configuration comprises:
(i) an ODH unit wherein a stream comprising ethane is subjected to ODH conditions, resulting in a stream comprising unconverted ethane, ethylene and water;
(ii) a water condensation unit wherein water is removed from a stream comprising unconverted ethane, ethylene and water by condensation, resulting in a stream comprising unconverted ethane and ethylene.

Further, in the present invention, the ODH configuration may comprise a carbon dioxide removal unit and a drying unit.

In the present invention, the integration between the above-mentioned steam cracker configuration and ODH configuration is effected by feeding an effluent coming from the ODH configuration, which effluent comprises unconverted ethane and ethylene, to the steam cracker configuration. Suitably, in the present invention, an effluent coming from the ODH configuration, which effluent comprises unconverted ethane and ethylene, is fed to the steam cracker configuration at a position which is downstream of the steam cracker unit.

WO2014134703 discloses incorporating an ethane ODH process into the work-up process as carried out in the downstream section of an ethane steam cracker configuration, wherein the ODH unit becomes part of the steam cracker configuration and the ethane containing feedstream to the ODH unit only originates from the steam cracker configuration.

In Figure 4 of WO2014134703, a C2 splitter for separating ethylene from ethane, which splitter is part of a steam cracker configuration, is connected with an ODH unit at the bottom of the C2 splitter. That is to say, in the process of said Figure 4, ethane from the bottom of the C2 splitter is sent to the ODH unit which is downstream of the C2 splitter. In the process of Figure 6 of WO2014134703, ethane and ethylene from the middle of the C2 splitter are sent to an ODH unit which is downstream of the C2 splitter. In the process of Figure 8 of WO2014134703, ethane and ethylene from hydrogenation units, which are part of a steam cracker configuration, are sent to an ODH unit which is downstream of the hydrogenation units. In all of these processes of Figures 4, 6 and 8 of WO2014134703, no effluent from the ODH unit is sent to the steam cracker configuration at any position.

Further, in Figure 7 of WO2014134703, ethane and ethylene from a steam cracker are sent to an ODH unit, the outlet stream of which is sent to a C2 splitter for separating ethylene from ethane, which splitter is part of the steam cracker configuration. That is to say, in the process of said Figure 7, ethane and ethylene from the steam cracker are sent to the ODH unit which is upstream of the C2 splitter. In the process of Figure 7 of WO2014134703, the ODH unit is part of the steam cracker configuration. There is no separate ODH configuration. For the inlet stream to the ODH unit originates from a steam cracker and the outlet stream from the ODH unit is sent a C2 splitter, both of which steam cracker and C2 splitter are part of the steam cracker configuration.

In all of the above-mentioned processes of Figures 4, 6, 7 and 8 of WO2014134703, the ODH unit as incorporated in a steam cracker configuration cannot be instrumental in enabling full use of the entire capacity of the downstream section of a steam cracker configuration, in a situation wherein the capacity of a steam cracker unit upstream of said downstream section does not match the higher capacity of the downstream section. For the streams which are fed to the ODH units in said Figures 4, 6, 7 and 8 originate, either directly or indirectly, from the effluent from the steam cracker unit. As stated in WO2014134703 (see page 46), the invention of WO2014134703 resides in converting (yet unconverted) ethane from the cracker product stream to ethylene in a cooperating ODH unit, with the objective of reducing the ethane load on the separation train. This is different from the present invention, wherein a larger amount of effluent, that is to say both effluent originating from the steam cracker unit and effluent originating from the ODH unit, can be fed to the downstream section of a steam cracker configuration, as compared to other cases wherein the amount of effluent that can be fed to such downstream section of a steam cracker configuration can only be determined by effluent originating from the steam cracker unit. Examples of such other cases are (i) cases wherein no ODH unit is used at all; or (ii) the above-described cases, as illlustrated in by the processes of Figures 4, 6, 7 and 8 of WO2014134703, wherein an ethane ODH process is incoporated into the work-up process as carried out in the downstream section of an ethane steam cracker configuration, wherein the ODH unit becomes part of the steam cracker configuration and the ethane containing feedstream to the ODH unit only originates from the steam cracker configuration.

Therefore, the present invention provides a process for the production of ethylene from ethane, involving steam cracking of ethane into ethylene and hydrogen, which process may be more technically advantageous, more efficient and more affordable as compared to other cases, as described above. Such technically advantageous process may preferably result in a lower energy demand and/or lower capital expenditure.

In particular, by integrating an ODH configuration with a steam cracker configuration through feeding an effluent coming from the ODH configuration, which effluent comprises unconverted ethane and ethylene, to the steam cracker configuration, in particular to the downstream section of the steam cracker configuration by feeding said effluent to the steam cracker configuration at a position which is downstream of the steam cracker unit, the present invention is useful for debottlenecking an existing steam cracker configuration or expanding the total capacity of a steam cracker configuration. Advantageously, in this way, a relatively high capacity of the downstream section of an ethane steam cracker configuration, as compared to the capacity of a steam cracker unit (comprising one or more furnaces) upstream of that downstream section, may be fully utilized. The foregoing may involve a case where a steam cracker furnace is close to or at the end of its life time. Further, this may involve a case where there is a desire to utilize the potential spare capacity in the back-end separation section of an existing steam cracker configuration because of additional design margin (overdesign of distillation towers or installation of higher capacity internals) and/or lower performance of the steam cracking furnace(s) than designed. The latter may also be a result of revamping an existing liquid steam cracker configuration to a gas (ethane) steam cracker configuration. Still further, this may apply to situations wherein 1 or more of a multitude of steam crackers has/have to be taken out of service (e.g. for maintenance or for any other reason). The foregoing examples show that the integration of the present invention may be advantageously applied in many practical situations, leading to a technically advantageous, efficient and affordable integrated process for the production of ethylene from ethane, involving both steam cracking of ethane into ethylene and hydrogen and oxidative dehydrogenation of ethane into ethylene and water.

In addition to enabling full utilization of the relatively high capacity of the downstream section of an ethane steam cracker configuration as described above, the present invention also has the following additional advantages. An ethane ODH configuration, comprising an ODH unit (e.g. 1 ODH reactor) and a water condensation unit, involves a low footprint (less physical area needed), a low capital intensity, a low energy intensity and consequently a low overall CO₂ emissions intensity. In an ethane ODH process, less energy is needed in compressors and distillation towers, because the ODH off-gas is of higher molecular weight (ODH effluent has substantially no light components such as hydrogen and methane produced, as compared to for example ethane steam cracking), the ODH process may be operated at a high pressure (e.g. 2-10 bar) and finally it is an exothermic chemical process producing net high pressure steam, which steam can also advantageously be used in the chemical complex of the present invention. In respect of the latter, steam produced in the ODH process may advantageously be used in the steam cracker configuration of the chemical complex. And vice versa: any steam produced in the steam cracking process may advantageously be used in the ODH configuration of the chemical complex. Further, generally, an ODH process produces much more concentrated product slate distribution (i.e. no or less by-products, like methane and C3+ hydrocarbons as produced in ethane steam cracking), but yet ODH effluents are still chemically compatible with ethane steam cracker effluents, making the line-up and separation requirement much simpler and less capital and energy intensive.

These and more advantages of the present invention will also be apparent from the following detailed description.

Within the present specification, the following terms have the following meanings.

"C3+ hydrocarbons" comprise hydrocarbons having a carbon number of 3 or higher. C3+ hydrocarbons may comprise propane and/or propylene.

"C2+ hydrocarbons" comprise hydrocarbons having a carbon number of 2 or higher. C2+ hydrocarbons may comprise ethane, ethylene, acetylene, propane and/or propylene.

"C2 hydrocarbons" comprise hydrocarbons having a carbon number of 2. C2 hydrocarbons may comprise ethane, ethylene and/or acetylene.

In respect of the process of the present invention, "steam cracker unit" means a unit wherein ethane is converted, by subjecting it to steam cracking conditions, into ethylene and hydrogen. In respect of the complex of the present invention, "steam cracker unit" means a unit which is suitable for converting ethane, by subjecting it to steam cracking conditions, into ethylene and hydrogen. The steam cracker unit may comprise a furnace.

In respect of the process of the present invention, "oxidative dehydrogenation unit" means a unit wherein ethane is converted, by subjecting it to oxidative dehydrogenation (ODH) conditions, into ethylene and water. In respect of the complex of the present invention, "oxidative dehydrogenation unit" means a unit which is suitable for converting, by subjecting it to ODH conditions, into ethylene and water. The ODH unit may comprise a reactor, which may be a catalytic reactor which is a reactor that contains a catalyst.

In respect of the process of the present invention, "carbon dioxide removal unit" means a unit wherein carbon dioxide is removed from a stream comprising ethylene, hydrogen and carbon dioxide. In respect of the complex of the present invention, "carbon dioxide removal unit" means a unit which is suitable for removing carbon dioxide from a stream comprising ethylene, hydrogen and carbon dioxide. Carbon dioxide removal agent as fed to the carbon dioxide removal unit may be an aqueous solution of a base, for example sodium hydroxide or an amine.

In respect of the process of the present invention, "drying unit" means a unit wherein water is removed from a stream comprising ethylene, hydrogen and water. In respect of the complex of the present invention, "drying unit" means a unit which is suitable for removing water from a stream comprising ethylene, hydrogen and water.

In respect of the process of the present invention, "acetylene hydrogenation unit" means a unit wherein acetylene, by subjecting it to hydrogenation conditions, is converted into ethylene. In respect of the complex of the present invention, "acetylene hydrogenation unit" means a unit which is suitable for converting acetylene, by subjecting it to hydrogenation conditions, into ethylene. The acetylene hydrogenation unit may comprise a reactor, which may be a catalytic reactor which is a reactor that contains a catalyst.

In respect of the process of the present invention, "C2 separation unit" means a unit wherein ethylene is separated from ethane. In respect of the complex of the present invention, "C2 separation unit" means a unit which is suitable for separating ethylene from ethane. Ethylene may be separated from ethane in any way, for example by means of distillation, absorption or adsorption.

Further, while the process and the chemical complex and configurations of the present invention and the stream or streams used in said process are described in terms of "comprising", "containing" or "including" one or more various described steps or units or components, they can also "consist essentially of" or "consist of" said one or more various described steps or units or components.

In the context of the present invention, in a case where a stream comprises two or more components, these components are to be selected in an overall amount not to exceed 100 vol.% or 100 wt.%.

The present process is a process for the production of ethylene from ethane, comprising:
subjecting a stream comprising ethane to steam cracking conditions in a steam cracker unit which is part of a steam cracker configuration, resulting in a stream comprising unconverted ethane, ethylene and hydrogen;
feeding a stream comprising unconverted ethane, ethylene and hydrogen to a separation unit which is part of the steam cracker configuration, and separating said stream in said separation unit into a stream comprising hydrogen and a stream comprising unconverted ethane and ethylene;
feeding a stream comprising unconverted ethane and ethylene to a C2 separation unit which is part of the steam cracker configuration, and separating said stream in the C2 separation unit into a stream comprising ethylene and a stream comprising unconverted ethane;
optionally recycling unconverted ethane from the stream comprising unconverted ethane coming from the C2 separation unit to the steam cracker unit;
subjecting a stream comprising ethane to oxidative dehydrogenation (ODH) conditions in an ODH unit which is part of an ODH configuration, resulting in a stream comprising unconverted ethane, ethylene and water;
feeding a stream comprising unconverted ethane, ethylene and water to a water condensation unit which is part of the ODH configuration, and removing water from said stream by condensation in the water condensation unit, resulting in a stream comprising unconverted ethane and ethylene;
feeding an effluent coming from the ODH configuration, which effluent comprises unconverted ethane and ethylene, to the steam cracker configuration;
optionally recycling unconverted ethane from the stream comprising unconverted ethane coming from the C2 separation unit to the ODH unit.

As described above, in the present invention, the integration between the steam cracker configuration and ODH configuration is effected by feeding an effluent coming from the ODH configuration, which effluent comprises unconverted ethane and ethylene, to the steam cracker configuration, suitably at a position which is downstream of the steam cracker unit. Further, suitably, no unconverted ethane from an effluent coming from the steam cracker configuration is fed to the ODH unit of the ODH configuration, with the exception of an optional recycle of unconverted ethane from an effluent coming from the C2 separation unit of the steam cracker configuration to the ODH unit of the ODH configuration. Still further, suitably, ethane is fed to the ODH unit which originates from one or more sources selected from the group consisting of a) the source of fresh ethane that is used to feed ethane to the steam cracker unit; b) another source of fresh ethane, other than said first source mentioned under a); and c) unconverted ethane from an effluent coming from the C2 separation unit. Within the present specification, by "fresh ethane" reference is made to ethane which does not comprise unconverted ethane. Within the present specification, by "unconverted ethane" reference is made to ethane that was subjected either to steam cracking conditions or to oxidative dehydrogenation conditions in the process of the present invention, but which was not converted. Regarding said sources of fresh ethane mentioned under a) and b) above, fresh ethane to be subjected to steam cracking conditions and/or to oxidative dehydrogenation conditions in the process of the present invention, may be provided by a plant which produces an ethane containing stream, either as a main stream or as a sidestream, such as a natural gas production plant, shale gas production plant, natural gas processing plant, Natural Gas Liquids (NGL) recovery and fractionation plant, Liquefied Natural Gas (LNG) production plant and so on, which plants may also be generally referred to as so-called "midstream" plants. Therefore, the present process may be integrated with any one of such midstream plants. However, in the present invention, the origin of fresh ethane is not essential.

Generally, in the present invention, the feed to the ODH unit may comprise: 1) unconverted ethane and no fresh ethane; or 2) unconverted ethane and fresh ethane; or 3) fresh ethane and no unconverted ethane. Likewise, generally, in the present invention, the feed to the steam cracker unit may comprise: 1) unconverted ethane and no fresh ethane; or 2) unconverted ethane and fresh ethane; or 3) fresh ethane and no unconverted ethane. In particular, fresh ethane may be fed to the ODH unit. Further, in particular, fresh ethane may be fed to the steam cracker unit. Still further, in particular, fresh ethane may be fed both to the ODH unit and to the steam cracker unit. As described above, feeding fresh ethane implies feeding ethane that was not subjected to steam cracking conditions and neither to oxidative dehydrogenation (ODH) conditions. Fresh ethane as fed to the ODH unit and fresh ethane as fed to the steam cracker unit may originate from the same source or different sources.

In the present process, the effluent coming from the ODH configuration, which effluent comprises unconverted ethane and ethylene and which effluent is fed to the steam cracker configuration, may be the stream comprising unconverted ethane and ethylene coming from the water condensation unit of the ODH configuration. Preferably, unconverted ethane and ethylene from the stream comprising unconverted ethane and ethylene coming from the water condensation unit of the ODH configuration are fed to the C2 separation unit of the steam cracker configuration.

In the present process, the stream coming from the steam cracker unit of the steam cracker configuration may comprise unconverted ethane, ethylene, hydrogen, methane, carbon monoxide, carbon dioxide and C3+ hydrocarbons; and the stream coming from the ODH unit of the ODH configuration may comprise unconverted ethane, ethylene, water, carbon monoxide and carbon dioxide. In such case, the ethylene production process of the present invention may comprise the following steps:
subjecting a stream comprising ethane to steam cracking conditions in a steam cracker unit which is part of a steam cracker configuration, resulting in a stream comprising unconverted ethane, ethylene, hydrogen, methane, carbon monoxide, carbon dioxide and C3+ hydrocarbons;
feeding a stream comprising unconverted ethane, ethylene, hydrogen, methane, carbon monoxide, carbon dioxide and C3+ hydrocarbons to a carbon dioxide removal unit which is part of the steam cracker configuration, and removing carbon dioxide from said stream in said carbon dioxide removal unit resulting in a stream comprising unconverted ethane, ethylene, hydrogen, methane, carbon monoxide and C3+ hydrocarbons;
feeding a stream comprising unconverted ethane, ethylene, hydrogen, methane, carbon monoxide and C3+ hydrocarbons to a separation unit which is part of the steam cracker configuration, and separating said stream in said separation unit into a stream comprising hydrogen, methane and carbon monoxide and a stream comprising C3+ hydrocarbons;
feeding unconverted ethane and ethylene from a stream comprising unconverted ethane, ethylene, hydrogen, methane, carbon monoxide and C3+ hydrocarbons to a C2 separation unit which is part of the steam cracker configuration, and separating said stream in the C2 separation unit into a stream comprising ethylene and a stream comprising unconverted ethane;
optionally recycling unconverted ethane from the stream comprising unconverted ethane coming from the C2 separation unit to the steam cracker unit;
subjecting a stream comprising ethane to oxidative dehydrogenation (ODH) conditions in an ODH unit which is part of an ODH configuration, resulting in a stream comprising unconverted ethane, ethylene, water, carbon monoxide and carbon dioxide;
feeding a stream comprising unconverted ethane, ethylene, water, carbon monoxide and carbon dioxide to a water condensation unit which is part of the ODH configuration, and removing water from said stream by condensation in the water condensation unit, resulting in a stream comprising unconverted ethane, ethylene, carbon monoxide and carbon dioxide;
feeding an effluent coming from the ODH configuration, which effluent comprises unconverted ethane and ethylene, to the steam cracker configuration;
optionally recycling unconverted ethane from the stream comprising unconverted ethane coming from the C2 separation unit to the ODH unit.

Further, the stream coming from the ODH unit of the ODH configuration may also comprise unconverted oxygen (used as an oxidizing agent in the ODH process). Preferably, such unconverted oxygen is removed before an effluent coming from the ODH configuration, which effluent comprises unconverted ethane and ethylene, is fed to the steam cracker configuration, in view of safety considerations and the presence of hydrogen in steam cracker effluents.

In the carbon dioxide removal step of the above-described process, a stream comprising unconverted ethane, ethylene, hydrogen, methane, carbon monoxide, carbon dioxide and C3+ hydrocarbons is fed to the carbon dioxide removal unit of the steam cracker configuration. The latter stream may be the stream coming from the steam cracker unit. Optionally, in case the stream comprising unconverted ethane, ethylene, hydrogen, methane, carbon monoxide and C3+ hydrocarbons, resulting from the carbon dioxide removal step, comprises water, the stream is fed to a drying unit as part of the steam cracker configuration in order to remove the water.

Further, in the above-described process, a stream comprising unconverted ethane, ethylene, hydrogen, methane, carbon monoxide and C3+ hydrocarbons is fed to a separation unit of the steam cracker configuration. The latter stream may be the stream coming from the above-mentioned carbon dioxide removal unit or optional drying unit. In the latter separation unit, the latter stream is separated into a stream comprising hydrogen, methane and carbon monoxide and a stream comprising C3+ hydrocarbons. In a 1^{st} embodiment, said stream comprising unconverted ethane, ethylene, hydrogen, methane, carbon monoxide and C3+ hydrocarbons is separated into a stream comprising hydrogen, methane and carbon monoxide and a stream comprising C2+ hydrocarbons, which C2+ hydrocarbons comprise unconverted ethane, ethylene and C3+ hydrocarbons. In a 2^{nd} embodiment, said stream comprising unconverted ethane, ethylene, hydrogen, methane, carbon monoxide and C3+ hydrocarbons is separated into a stream comprising hydrogen, methane, carbon monoxide and C2 hydrocarbons, which C2 hydrocarbons comprise unconverted ethane and ethylene, and a stream comprising C3+ hydrocarbons. In said 1^{st} embodiment, the separated stream comprising C2+ hydrocarbons may be fed to a further separation unit, wherein said stream is separated into a stream comprising C2 hydrocarbons and a stream comprising C3+ hydrocarbons. In said 2^{nd} embodiment, the separated stream comprising hydrogen, methane, carbon monoxide and C2 hydrocarbons may be fed to a further separation unit, wherein said stream is separated into a stream comprising hydrogen, methane and carbon monoxide and a stream comprising C2 hydrocarbons. In both said embodiments, the separated stream comprising C2 hydrocarbons coming from said further separation unit may be fed to the above-mentioned C2 separation unit of the steam cracker configuration, wherein the latter stream is separated into a stream comprising ethylene and a stream comprising unconverted ethane.

Still further, in the above-described process, the stream comprising unconverted ethane, ethylene, carbon monoxide and carbon dioxide coming from the water condensation unit of the ODH configuration may be fed to a carbon dioxide removal unit which is part of the ODH configuration, wherein carbon dioxide is removed from said stream resulting in a stream comprising unconverted ethane, ethylene and carbon monoxide. Optionally, in case the stream comprising unconverted ethane, ethylene and carbon monoxide, resulting from the carbon dioxide removal step, comprises water, the stream may be fed to a drying unit as part of the ODH configuration in order to remove the water.

Still further, in the above-described process, an effluent coming from the ODH configuration, which effluent comprises unconverted ethane and ethylene, is fed to the steam cracker configuration. Suitably, said effluent coming from the ODH configuration is an effluent originating, either directly or indirectly, from the ODH unit.

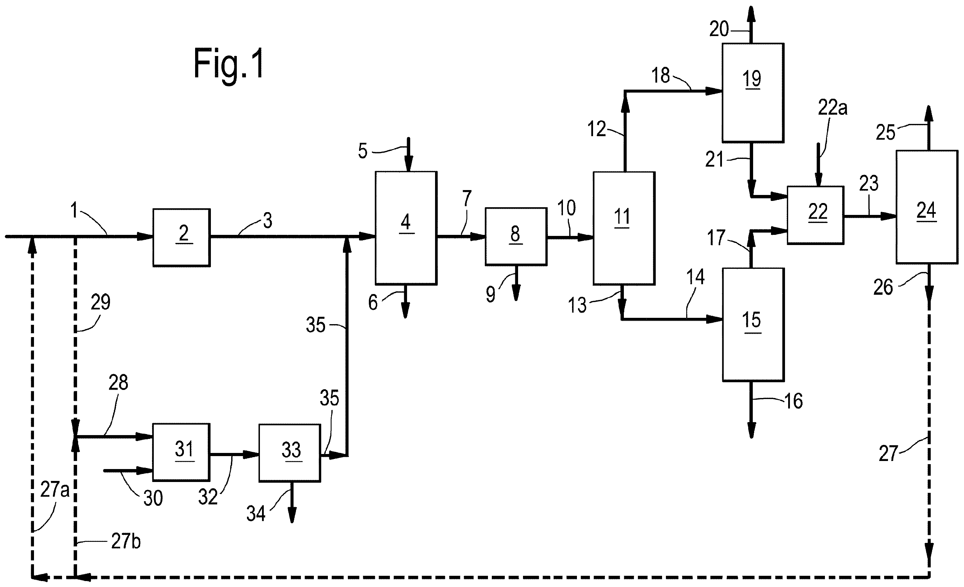

In the above-described process, the above-mentioned effluent coming from the ODH configuration, which effluent comprises unconverted ethane and ethylene and which effluent is fed to the steam cracker configuration, may be the stream comprising unconverted ethane, ethylene, carbon monoxide and carbon dioxide coming from the water condensation unit of the ODH configuration. Preferably, the latter stream comprising unconverted ethane, ethylene, carbon monoxide and carbon dioxide coming from the water condensation unit of the ODH configuration is fed to the carbon dioxide removal unit of the steam cracker configuration. This is illustrated in Figure 1 (by line 35).

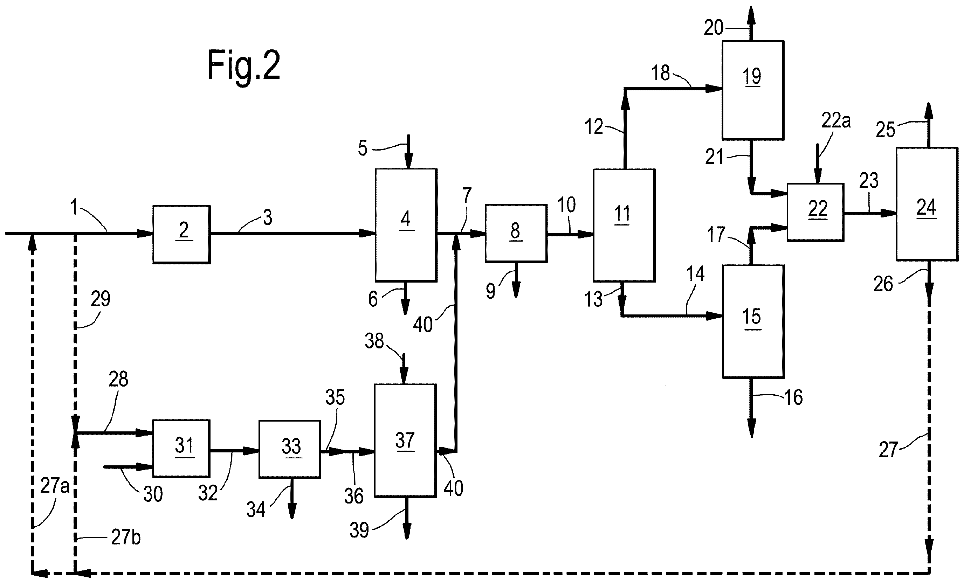

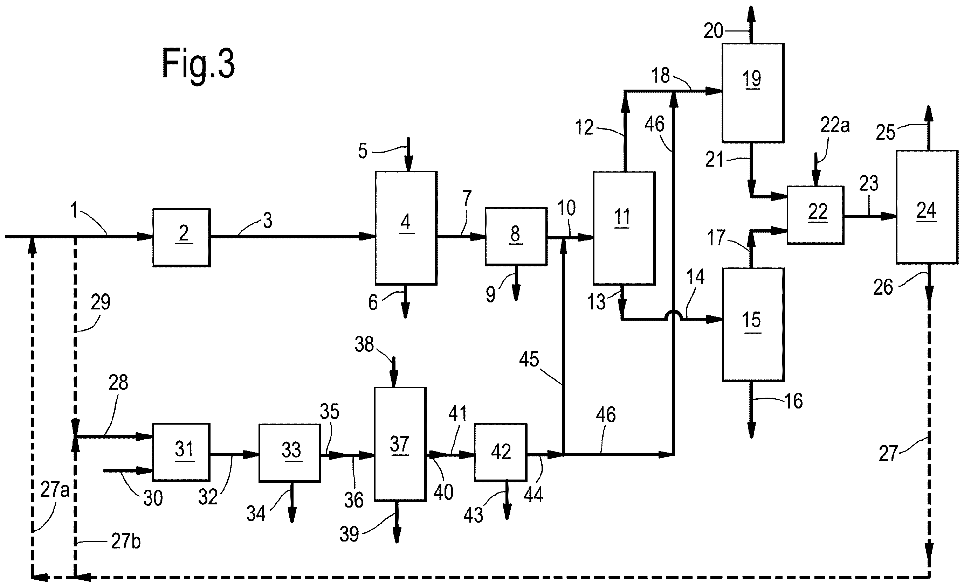

Further, in the above-described process, the above-mentioned effluent coming from the ODH configuration, which effluent comprises unconverted ethane and ethylene and which effluent is fed to the steam cracker configuration, may be the stream comprising unconverted ethane, ethylene and carbon monoxide coming from the optional carbon dioxide removal unit of the ODH configuration. Preferably, the latter stream comprising unconverted ethane, ethylene and carbon monoxide coming from the optional carbon dioxide removal unit of the ODH configuration is fed to the separation unit of the steam cracker configuration, wherein a stream comprising unconverted ethane, ethylene, hydrogen, methane, carbon monoxide and C3+ hydrocarbons is separated into a stream comprising hydrogen, methane and carbon monoxide and a stream comprising C3+ hydrocarbons. In a case wherein the streams resulting from the carbon dioxide removal steps comprise water, as described above, the stream coming from the carbon dioxide removal unit of the ODH configuration may be fed to the above-mentioned drying unit of the steam cracker configuration. This is illustrated in Figure 2 (by line 40). Further, in a case wherein the streams resulting from the carbon dioxide removal steps comprise water and the ODH configuration comprises a drying unit, as described above, the stream coming from the drying unit of the ODH configuration and comprising unconverted ethane, ethylene and carbon monoxide may be fed to that separation unit of the steam cracker configuration, in which separation unit a stream comprising unconverted ethane, ethylene, hydrogen, methane, carbon monoxide and C3+ hydrocarbons is separated into a stream comprising hydrogen, methane and carbon monoxide and a stream comprising C2+ hydrocarbons (above-described "1^{st} embodiment"). This is illustrated in Figure 3 (by line 45). Further, in a case wherein the streams resulting from the carbon dioxide removal steps comprise water and the ODH configuration comprises a drying unit, as described above, the stream coming from the drying unit of the ODH configuration and comprising unconverted ethane, ethylene and carbon monoxide may be fed to that separation unit of the steam cracker configuration, in which separation unit a stream comprising hydrogen, methane, carbon monoxide and C2 hydrocarbons is separated into a stream comprising hydrogen, methane and carbon monoxide and a stream comprising C2 hydrocarbons (above-described "2^{nd} embodiment"). This is also illustrated in Figure 3 (by line 46).

In the present process, the stream coming from the steam cracker unit of the steam cracker configuration may additionally comprise acetylene; and the stream coming from the ODH unit of the ODH configuration may additionally comprise acetylene. In such case, the ethylene production process of the present invention may comprise an acetylene hydrogenation step wherein in an acetylene hydrogenation unit a stream comprising unconverted ethane, ethylene and acetylene is subjected to hydrogenation conditions, so as to convert acetylene into ethylene. Preferably, the acetylene hydrogenation unit is part of the steam cracker configuration. In the latter case, a stream comprising unconverted ethane, ethylene and acetylene may be fed to the acetylene hydrogenation unit of the steam cracker configuration, resulting in a stream comprising unconverted ethane and ethylene which latter stream may be fed to the above-mentioned C2 separation unit. In such case, a stream containing hydrogen may be fed to the acetylene hydrogenation unit of the steam cracker configuration. Hydrogen is a hydrogenation agent which may hydrogenate acetylene into ethylene and which may be derived from the steam cracker configuration.

More in particular, in the present process in a case where acetylene is present as described above, in the above-mentioned 1^{st} embodiment, a stream comprising unconverted ethane, ethylene, acetylene, hydrogen, methane, carbon monoxide and C3+ hydrocarbons is separated into a stream comprising hydrogen, methane and carbon monoxide and a stream comprising C2+ hydrocarbons, which C2+ hydrocarbons comprise unconverted ethane, ethylene, acetylene and C3+ hydrocarbons. Further, in the above-mentioned 2^{nd} embodiment, a stream comprising unconverted ethane, ethylene, acetylene, hydrogen, methane, carbon monoxide and C3+ hydrocarbons is separated into a stream comprising hydrogen, methane, carbon monoxide and C2 hydrocarbons, which C2 hydrocarbons comprise unconverted ethane, ethylene and acetylene, and a stream comprising C3+ hydrocarbons. In said 1^{st} embodiment, the separated stream comprising C2+ hydrocarbons may be fed to a further separation unit, wherein said stream is separated into a stream comprising C2 hydrocarbons and a stream comprising C3+ hydrocarbons. In said 2^{nd} embodiment, the separated stream comprising hydrogen, methane, carbon monoxide and C2 hydrocarbons may be fed to a further separation unit, wherein said stream is separated into a stream comprising hydrogen, methane and carbon monoxide and a stream comprising C2 hydrocarbons. In both said embodiments, the separated stream comprising C2 hydrocarbons coming from said further separation unit may be fed to an acetylene hydrogenation unit as part of the steam cracker configuration, wherein acetylene is converted into ethylene, resulting in a stream comprising unconverted ethane and ethylene, which latter stream may then be fed to the above-mentioned C2 separation unit of the steam cracker configuration, wherein the latter stream is separated into a stream comprising ethylene and a stream comprising unconverted ethane.

In the process of the present invention, a stream comprising ethane is subjected to steam cracking conditions in a steam cracker unit which is part of a steam cracker configuration, resulting in a stream comprising unconverted ethane, ethylene and hydrogen. Suitable steam cracking conditions for this steam cracking step are described hereinbelow.

Suitably, in the above-mentioned steam cracking step, no oxygen containing stream is fed to the steam cracker unit since there is no need to use oxygen as an oxidizing agent. However, carbon dioxide may still be produced as an impurity in the presence of oxygen (resulting from some small air ingress into the steam cracker unit) and/or in water-shift reactions of hydrocarbons. Further, carbon monoxide and carbon dioxide may enter the steam cracking process as contaminants in the feed.

Further, suitably, in the above-mentioned steam cracking step, no catalyst is used. Preferably, said steam cracking step is performed at an elevated temperature, more preferably in the range of from 650 to 1000 °C, most preferably of from 750 to 950 °C. Hydrocarbon steam cracking processes are well known. Reference is for instance made to Kniel et al., Ethylene, Keystone to the petrochemical industry, Marcel Dekker, Inc, New York, 1980, in particular chapter 6 and 7.

In the process of the present invention, a stream comprising ethane is subjected to oxidative dehydrogenation (ODH) conditions in an ODH unit which is part of an ODH configuration, resulting in a stream comprising unconverted ethane, ethylene and water. Suitable ODH conditions for this ODH step are described hereinbelow.

In the above-mentioned ODH step, subjecting a stream comprising ethane to ODH conditions may comprise contacting the stream comprising ethane with oxygen (O₂). Said oxygen is the oxidizing agent in the ODH reaction. In the ODH step, oxygen (O₂) and a stream comprising ethane may be fed to the ODH unit. The ODH unit may comprise a reactor, which reactor may contain an ODH catalyst, in particular a mixed metal oxide catalyst containing molybdenum, vanadium, niobium and optionally tellurium. Oxygen and ethane are then contacted with said catalyst in the ODH reactor, resulting in oxidative dehydrogenation of the ethane.

In the ODH step of the process of the present invention, oxygen and ethane may be fed to the reactor together or separately. That is to say, one or more feed streams, suitably gas streams, comprising one or more of said 2 components may be fed to the reactor. For example, one feed stream comprising oxygen and ethane may be fed to the reactor. Alternatively, two or more feed streams, suitably gas streams, may be fed to the reactor, which feed streams may form a combined stream inside the reactor. For example, one feed stream comprising oxygen and another feed stream comprising ethane may be fed to the reactor separately.

Further, in the ODH step of the process of the present invention, suitably during contacting oxygen and ethane with an ODH catalyst, the temperature is of from 300 to 500 °C. More preferably, said temperature is of from 310 to 450 °C, more preferably of from 320 to 420 °C, most preferably of from 330 to 420 °C.

Still further, in the above-mentioned ODH step, suitably during contacting the oxygen and ethane with an ODH catalyst, typical pressures are 0.1-30 or 0.1-20 bara (i.e. "bar absolute"). Further, preferably, said pressure is of from 0.1 to 15 bara, more preferably of from 1 to 8 bara, most preferably of from 3 to 8 bara.

The product of the above-mentioned ODH step comprises the dehydrogenated equivalent of ethane, that is to say ethylene. Ethylene is initially formed in said step. However, in said same step, ethylene may be further oxidized under the same conditions into the corresponding carboxylic acid, that is to say acetic acid.

In addition to oxygen and ethane, an inert gas may also be fed to the ODH reactor. Said inert gas may be selected from the group consisting of the noble gases and nitrogen (N₂). Preferably, the inert gas is nitrogen or argon, more preferably nitrogen. Said oxygen is an oxidizing agent, thereby resulting in oxidative dehydrogenation of ethane. Said oxygen may originate from any source, such as for example air. Ranges for the molar ratio of oxygen to ethane which are suitable, are of from 0.01 to 1, more suitably 0.05 to 0.5. Said ratio of oxygen to ethane is the ratio before oxygen and ethane are contacted with the catalyst. In other words, said ratio of oxygen to ethane is the ratio of oxygen as fed to ethane as fed. Obviously, after contact with the catalyst, at least part of the oxygen and ethane gets consumed.

Preferably, in the ODH step of the process of the present invention, the ODH catalyst is a heterogeneous catalyst. Further, preferably, the ODH catalyst is a mixed metal oxide catalyst containing molybdenum, vanadium, niobium and optionally tellurium as the metals, which catalyst may have the following formula:

Mo₁VₐTe_{b}Nb_{c}Oₙ

wherein:
a, b, c and n represent the ratio of the molar amount of the element in question to the molar amount of molybdenum (Mo) ;
a (for V) is from 0.01 to 1, preferably 0.05 to 0.60, more preferably 0.10 to 0.40, more preferably 0.20 to 0.35, most preferably 0.25 to 0.30;
b (for Te) is 0 or from >0 to 1, preferably 0.01 to 0.40, more preferably 0.05 to 0.30, more preferably 0.05 to 0.20, most preferably 0.09 to 0.15;
c (for Nb) is from >0 to 1, preferably 0.01 to 0.40, more preferably 0.05 to 0.30, more preferably 0.10 to 0.25, most preferably 0.14 to 0.20; and
n (for O) is a number which is determined by the valency and frequency of elements other than oxygen.

The amount of the catalyst in the above-mentioned ODH step is not essential. Preferably, a catalytically effective amount of the catalyst is used, that is to say an amount sufficient to promote the ethane oxydehydrogenation reaction.

The ODH reactor that may be used in the above-mentioned ODH step may be any reactor, including fixed-bed and fluidized-bed reactors. Suitably, the reactor is a fixed-bed reactor.

Examples of oxydehydrogenation processes, including catalysts and process conditions, are for example disclosed in above-mentioned US7091377, WO2003064035, US20040147393, WO2010096909 and US20100256432, the disclosures of which are herein incorporated by reference.

Water is formed during the ethane ODH reaction that takes place in the ODH step of the present process. In the process of the present invention, a stream comprising unconverted ethane, ethylene and water, suitably the stream comprising unconverted ethane, ethylene and water resulting from said ODH step, is fed to a water condensation unit which is part of the ODH configuration, and water is removed from said stream by condensation in the water condensation unit, resulting in a stream comprising unconverted ethane and ethylene. In said water condensation step, water in the stream comprising unconverted ethane, ethylene and water may easily be condensed by cooling down the latter stream to a lower temperature, for example room temperature, after which the condensed water can be separated from the stream comprising unconverted ethane, ethylene and water.

In the present invention, any carbon dioxide may be removed from streams containing carbon dioxide by any one of well-known methods. As mentioned above, a suitable carbon dioxide removal agent that may be fed to a carbon dioxide removal unit may be an aqueous solution of a base, for example sodium hydroxide or an amine. After such carbon dioxide removal, the stream should be dried in a drying unit to remove residual water from the stream. Contacting an aqueous solution of an amine with a carbon dioxide containing stream is preferred in a case where the carbon dioxide amount is relatively high, for example in the case of an ethane ODH effluent. Contacting an aqueous solution of sodium hydroxide with a carbon dioxide containing stream is preferred in a case where the carbon dioxide amount is relatively low, for example 1) in the case of an ethane steam cracker effluent or 2) in the case of an ethane ODH effluent that was treated with an aqueous solution of an amine and which still contains some residual carbon dioxide. In the present invention, a carbon dioxide removal unit as part of the steam cracker configuration may comprise a subunit wherein carbon dioxide is removed by an aqueous solution of an amine and a downstream subunit wherein carbon dioxide is removed by an aqueous solution of sodium hydroxide. In the latter case, it may be preferred that carbon dioxide containing effluent from the water condensation unit of the ODH configuration is fed to the first subunit wherein carbon dioxide is removed by an aqueous solution of an amine. Further, a carbon dioxide removal unit as part of the steam cracker configuration may only comprise a unit wherein carbon dioxide is removed by an aqueous solution of sodium hydroxide. In the latter case, it may be preferred that carbon dioxide containing effluent from the water condensation unit of the ODH configuration is first fed to a carbon dioxide removal unit as part of the ODH configuration wherein carbon dioxide is removed by an aqueous solution of an amine, and then fed to the carbon dioxide removal unit of the steam cracker configuration wherein carbon dioxide is removed by an aqueous solution of sodium hydroxide.

Further, the present invention relates to a chemical complex which is suitable for performing the above-described ethylene production process of the present invention, more in particular a chemical complex which comprises a steam cracker configuration and an oxidative dehydrogenation (ODH) configuration, wherein:
the steam cracker configuration comprises a steam cracker unit, a carbon dioxide removal unit, an optional drying unit, a 1^{st} separation unit, a 2^{nd} separation unit, an optional acetylene hydrogenation unit and a C₂ separation unit, wherein each unit comprises one or more feed lines and one or more effluent lines;
the ODH configuration comprises an ODH unit, a water condensation unit, an optional carbon dioxide removal unit and an optional drying unit, wherein each unit comprises one or more feed lines and one or more effluent lines;
the ODH configuration is integrated with the steam cracker configuration at a position upstream of the optional acetylene hydrogenation unit and upstream of the C2 separation unit; and
no feed line of the ODH unit is integrated with a feed line or effluent line of a unit of the steam cracker configuration, with the exception that a feed line of the steam cracker unit and a feed line of the ODH unit may be integrated, and/or that an optional recycle effluent line of the C2 separation unit and a feed line of the ODH unit may be integrated.

Suitably, in the above-described chemical complex, the steam cracker configuration comprises the following units in the following order going from upstream to downstream: a steam cracker unit, a carbon dioxide removal unit, an optional drying unit, a 1^{st} separation unit, a 2^{nd} separation unit, an optional acetylene hydrogenation unit and a C₂ separation unit. Further, the steam cracker configuration may comprise a quench unit (quench tower) and/or 1 or more compression units (compressors).

Further, suitably, in the above-described chemical complex, the ODH configuration comprises the following units in the following order going from upstream to downstream: an ODH unit, a water condensation unit, an optional carbon dioxide removal unit and an optional drying unit. Further, the ODH configuration may comprise an oxygen removal unit and/or 1 or more compression units (compressors).

In a first embodiment of the above-described chemical complex, the 1^{st} separation unit is a separation unit which is suitable for separating a stream comprising C3+ hydrocarbons, C2 hydrocarbons, hydrogen, optionally carbon monoxide and optionally methane into a stream comprising hydrogen, optionally carbon monoxide and optionally methane and a stream comprising C2+ hydrocarbons; and the 2^{nd} separation unit is a separation unit which is suitable for separating a stream comprising C2+ hydrocarbons into a stream comprising C2 hydrocarbons and a stream comprising C3+ hydrocarbons.

In a second embodiment of the above-described chemical complex, the 1^{st} separation unit is a separation unit which is suitable for separating a stream comprising C3+ hydrocarbons, C2 hydrocarbons, hydrogen, optionally carbon monoxide and optionally methane into a stream comprising C2 hydrocarbons, hydrogen, optionally carbon monoxide and optionally methane and a stream comprising C3+ hydrocarbons; and the 2^{nd} separation unit is a separation unit which is suitable for separating a stream comprising C2 hydrocarbons, hydrogen, optionally carbon monoxide and optionally methane into a stream comprising hydrogen, optionally carbon monoxide and optionally methane and a stream comprising C2 hydrocarbons.

All features and embodiments as described above with respect to the steam cracker configuration and the ODH configuration, including their units, as used in the process of the present invention, also apply to the corresponding configurations and units in the chemical complex of the present invention.

In particular, in the chemical complex of the present invention, an effluent line of a unit of the ODH configuration may be integrated with a feed line or effluent line of a unit of the steam cracker configuration. Within the present specification, "integration" of a line from the ODH configuration with a line from the steam cracker configuration in the chemical complex of the present invention means that the two lines in question are connected.

In the chemical complex of the present invention, the effluent line of a unit of the ODH configuration that may be integrated with a feed line or effluent line of a unit of the steam cracker configuration, may be one or more of the following: a) an effluent line of the water condensation unit; b) an effluent line of the optional carbon dioxide removal unit; and c) an effluent line of the optional drying unit.

In the chemical complex of the present invention, an effluent line of the water condensation unit of the ODH configuration may be integrated with a feed line of the carbon dioxide removal unit of the steam cracker configuration.

In the chemical complex of the present invention, an effluent line of the optional carbon dioxide removal unit of the ODH configuration may be integrated with a feed line of the optional drying unit of the steam cracker configuration.

In the above-mentioned first embodiment of the chemical complex of the present invention, an effluent line of the optional drying unit of the ODH configuration may be integrated with a feed line of the 1^{st} separation unit of the steam cracker configuration.

In the above-mentioned second embodiment of the chemical complex of the present invention, an effluent line of the optional drying unit of the ODH configuration may be integrated with a feed line of the 2^{nd} separation unit of the steam cracker configuration.

The process and chemical complex of the present invention are further illustrated by Figures 1-3.

In Figure 1, a steam cracker configuration is shown. Said steam cracker configuration comprises steam cracker unit 2, carbon dioxide removal unit 4, drying unit 8, separation units 11, 15, 19 and 24 and acetylene hydrogenation unit 22. All of said separation units 11, 15, 19 and 24 are distillation columns. Further, in Figure 1, an oxidative dehydrogenation (ODH) configuration integrated with said steam cracker configuration is also shown. Said ODH configuration comprises ODH unit 31 and water condensation unit 33.

In said Figure 1, stream 1 comprising ethane is fed to steam cracker unit 2 operating under steam cracking conditions. Product stream 3 coming from steam cracker unit 2 comprises C3+ hydrocarbons, ethane, ethylene, acetylene, methane, hydrogen, carbon monoxide and carbon dioxide. Said stream 3 is fed to carbon dioxide removal unit 4. Carbon dioxide removal agent is fed to carbon dioxide removal unit 4 via stream 5. Said carbon dioxide removal agent may be an aqueous solution of a base, for example sodium hydroxide or an amine. Carbon dioxide removal unit 4 may comprise a subunit wherein carbon dioxide is removed by an aqueous solution of an amine and a downstream subunit wherein carbon dioxide is removed by an aqueous solution of sodium hydroxide. Carbon dioxide is removed via aqueous stream 6. Stream 7 coming from carbon dioxide removal unit 4, which comprises C3+ hydrocarbons, ethane, ethylene, acetylene, methane, hydrogen, carbon monoxide and water, is fed to drying unit 8. In drying unit 8, water is removed via stream 9. Stream 10 coming from drying unit 8, which comprises C3+ hydrocarbons, ethane, ethylene, acetylene, methane, hydrogen and carbon monoxide, is fed to separation unit 11.

In a 1^{st} embodiment, in separation unit 11, stream 10 comprising C3+ hydrocarbons, ethane, ethylene, acetylene, methane, hydrogen and carbon monoxide is separated into a top stream 12 comprising methane, hydrogen and carbon monoxide and a bottom stream 13 comprising C3+ hydrocarbons, ethane, ethylene and acetylene. In said 1^{st} embodiment, stream 13 is fed as stream 14 to separation unit 15. In separation unit 15, stream 14 is separated into a top stream 17 comprising ethane, ethylene and acetylene and a bottom stream 16 comprising C3+ hydrocarbons.

In a 2^{nd} embodiment, in separation unit 11, stream 10 comprising C3+ hydrocarbons, ethane, ethylene, acetylene, methane, hydrogen and carbon monoxide is separated into a top stream 12 comprising ethane, ethylene, acetylene, methane, hydrogen and carbon monoxide and a bottom stream 13 comprising C3+ hydrocarbons. In said 2^{nd} embodiment, stream 12 is fed as stream 18 to separation unit 19. In separation unit 19, stream 12 is separated into a top stream 20 comprising methane, hydrogen and carbon monoxide and a bottom stream 21 comprising ethane, ethylene and acetylene.

Stream 17 comprising ethane, ethylene and acetylene (above-mentioned 1^{st} embodiment) or stream 21 comprising ethane, ethylene and acetylene (above-mentioned 2^{nd} embodiment) is fed to acetylene hydrogenation unit 22. In acetylene hydrogenation unit 22, acetylene is hydrogenated using hydrogen stream 22a into ethylene resulting in stream 23 comprising ethane and ethylene. Said stream 23 is fed to separation unit 24 (a "C2 separation unit") wherein stream 23 is separated into a top stream 25 comprising ethylene and a bottom stream 26 comprising ethane.

Further, in said Figure 1, stream 28 comprising ethane and stream 30 comprising an oxidizing agent are fed to ODH unit 31 containing an ODH catalyst and operating under ODH conditions. The source of ethane as fed to steam cracker unit 2 and ODH unit 31 may be the same or different. In a case where the source is the same, ethane from stream 1 may be fed via stream 29 and stream 28 to ODH unit 31. Product stream 32 coming from ODH unit 28 comprises water, ethane, ethylene, acetylene, carbon monoxide, carbon dioxide and any acetic acid. Said stream 32 is fed to water condensation unit 33. In water condensation unit 33, water and any acetic acid are removed by condensation via stream 34. In Figure 1, stream 35 coming from water condensation unit 33, which comprises ethane, ethylene, acetylene, carbon monoxide and carbon dioxide, is fed to carbon dioxide removal unit 4 which is part of the steam cracker configuration. Additionally, before feeding stream 35 to carbon dioxide removal unit 4, stream 35 may first be fed to another carbon dioxide removal unit (not shown in Figure 1) which is not part of the steam cracker configuration, and wherein a relatively larger amount of carbon dioxide may be removed than in carbon dioxide removal unit 4, for example by using an aqueous solution of an amine as carbon dioxide removal agent. Generally, the effluent from an ODH unit contains relatively more carbon dioxide than the effluent from a steam cracker unit. In the latter case, carbon dioxide removal unit 4 may only comprise a unit wherein carbon dioxide is removed by an aqueous solution of sodium hydroxide.

Still further, in said Figure 1, ethane from stream 26 may be recycled via stream 27. Stream 27a coming from stream 27 and comprising ethane may be fed to steam cracker unit 2. Stream 27b coming from stream 27 and comprising ethane may be fed to ODH unit 31.

In Figure 2, a steam cracker configuration and an oxidative dehydrogenation (ODH) configuration integrated with said steam cracker configuration are shown. The steam cracker configuration is identical to that of Figure 1. In Figure 2, the ODH configuration comprises ODH unit 31, water condensation unit 33 and carbon dioxide removal unit 37.

The process of Figure 2 is the same as the process of Figure 1, with the exception that stream 35 coming from water condensation unit 33 which is part of the ODH configuration, which stream 35 comprises ethane, ethylene, acetylene, carbon monoxide and carbon dioxide, is not fed to carbon dioxide removal unit 4 which is part of the steam cracker configuration, but is fed, via stream 36, to carbon dioxide removal unit 37 which is part of the ODH configuration. Carbon dioxide removal agent is fed to carbon dioxide removal unit 37 via stream 38. Said carbon dioxide removal agent may be an aqueous solution of a base, for example sodium hydroxide or an amine. Carbon dioxide is removed via aqueous stream 39. In Figure 2, stream 40 coming from carbon dioxide removal unit 37, which comprises ethane, ethylene, acetylene, carbon monoxide and water, is fed to drying unit 8 which is part of the steam cracker configuration. In Figure 2, carbon dioxide removal unit 4 may only comprise a unit wherein carbon dioxide is removed by an aqueous solution of sodium hydroxide. Further, in Figure 2, carbon dioxide removal unit 37 may comprise a subunit wherein carbon dioxide is removed by an aqueous solution of an amine and a downstream subunit wherein carbon dioxide is removed by an aqueous solution of sodium hydroxide.

In Figure 3, a steam cracker configuration and an oxidative dehydrogenation (ODH) configuration integrated with said steam cracker configuration are shown. The steam cracker configuration is identical to that of Figures 1 and 2. In Figure 3, the ODH configuration comprises ODH unit 31, water condensation unit 33, carbon dioxide removal unit 37 and drying unit 42.

The process of Figure 3 is the same as the process of Figure 2, with the exception that stream 40 coming from carbon dioxide removal unit 37 which is part of the ODH configuration, which stream 40 comprises ethane, ethylene, acetylene and carbon monoxide, is not fed to drying unit 8 which is part of the steam cracker configuration, but is fed, via stream 41, to drying unit 42 which is part of the ODH configuration. In drying unit 42, water is removed via stream 43. Stream 44 coming from drying unit 42 comprises ethane, ethylene, acetylene and carbon monoxide. In a 1^{st} embodiment of Figure 3, which corresponds to the 1^{st} embodiment as described with respect to Figure 1, stream 44 is sent as stream 45 to separation unit 11 which is part of the steam cracker configuration. In a 2^{nd} embodiment of Figure 3, which corresponds to the 2^{nd} embodiment as described with respect to Figure 1, stream 44 is sent as stream 46 to separation unit 19 which is part of the steam cracker configuration.

In Figures 1-3, the steam cracker configuration may comprise one or more compressors (not shown in Figures 1-3). One compressor may be placed between steam cracker unit 2 and carbon dioxide removal unit 4. In Figure 1, said compressor may be placed between steam cracker unit 2 and the point at which streams 3 and 35 are combined. Further, another compressor may be placed between carbon dioxide removal unit 4 and drying unit 8. In Figure 2, said compressor may be placed between drying unit 8 and the point at which streams 7 and 40 are combined.

Further, in Figures 1-3, acetylene hydrogenation unit 22 which is part of the steam cracker configuration may be placed at another position within the steam cracker configuration. In Figure 1, acetylene hydrogenation unit 22 may be placed at any one of the following positions (not shown in Figure 1): 1) between carbon dioxide removal unit 4 and the point at which streams 3 and 35 are combined; 2) between carbon dioxide removal unit 4 and drying unit 8; 3) between drying unit 8 and separation unit 11; 4) between separation units 11 and 19 (only in 2^{nd} embodiment as described with respect to Figure 1). In Figure 2, acetylene hydrogenation unit 22 may be placed at any one of the following positions (not shown in Figure 2): 1) between drying unit 8 and the point at which streams 7 and 40 are combined; 2) between drying unit 8 and separation unit 11; 3) between separation units 11 and 19 (only in 2^{nd} embodiment as described with respect to Figure 1). In Figure 3, acetylene hydrogenation unit 22 may be placed at any one of the following positions (not shown in Figure 3): 1) between separation unit 11 and the point at which streams 10 and 45 are combined (only in 1^{st} embodiment as described with respect to Figure 1); 2) between separation unit 19 and the point at which streams 18 and 46 are combined (only in 2^{nd} embodiment as described with respect to Figure 1). In all of these cases, advantageously no separate hydrogen stream 22a needs to be fed to acetylene hydrogenation unit 22 as hydrogen is still present in said streams 3, 7, 10 and 18.

In a further embodiment (not shown in Figures 1-3), a feed comprising fresh ethane and optionally propane is introduced into the downstream section of the steam cracker configuration, in particular into line 10 to column 11 or into line 14 to column 15, by which any propane from the fresh ethane feed is advantageously removed together with any C3+ hydrocarbons originating from the steam cracker unit, thereby removing the need to use a separate, additional depropanizer. In said further embodiment, fresh ethane does not have to be fed directly to ODH reactor 31, but an ethane recycle from line 27 can be sufficient. Said ethane recycle from line 27 would still comprise fresh ethane, that is to say the fresh ethane as fed to column 11 or 15, which ethane was not subjected to steam cracking conditions and neither to oxidative dehydrogenation (ODH) conditions. Thus, fresh ethane as fed in the foregoing way (that is to say, indirectly via line 10 or line 14) to the ODH unit would thus originate from a source other than a source of fresh ethane that is used to feed ethane directly to the steam cracker unit. In addition, said ethane recycle from line 27 would comprise unconverted ethane, that is to say unconverted ethane originating from the steam cracker unit.

The present invention is also applicable to a process wherein instead of subjecting a stream comprising ethane to steam cracking conditions, a stream comprising naphtha is subjected to steam cracking conditions in the steam cracker unit which is part of the steam cracker configuration of the present invention. The above-discussed embodiments and preferences in relation to ethane steam cracking equally apply to the above case wherein a naphtha steam cracker configuration and an ODH configuration are integrated. Generally, the stream resulting from a naphtha steam cracker is subjected to a quench step wherein cooling and removal of heavies are performed. Such quench step may be included between units 2 and 4 in Figures 1-3.

## Claims

1. Process for the production of ethylene from naphtha and ethane, comprising:
subjecting a stream comprising naphtha to steam cracking conditions in a steam cracker unit which is part of a steam cracker configuration, resulting in a stream comprising unconverted ethane, ethylene and hydrogen;
feeding a stream comprising unconverted ethane, ethylene and hydrogen to a separation unit which is part of the steam cracker configuration, and separating said stream in said separation unit into a stream comprising hydrogen and a stream comprising unconverted ethane and ethylene;
feeding a stream comprising unconverted ethane and ethylene to a C2 separation unit which is part of the steam cracker configuration, and separating said stream in the C2 separation unit into a stream comprising ethylene and a stream comprising unconverted ethane;
optionally recycling unconverted ethane from the stream comprising unconverted ethane coming from the C2 separation unit to the steam cracker unit;
subjecting a stream comprising ethane to oxidative dehydrogenation (ODH) conditions in an ODH unit which is part of an ODH configuration, resulting in a stream comprising unconverted ethane, ethylene and water;
feeding a stream comprising unconverted ethane, ethylene and water to a water condensation unit which is part of the ODH configuration, and removing water from said stream by condensation in the water condensation unit, resulting in a stream comprising unconverted ethane and ethylene;
feeding an effluent coming from the ODH configuration, which effluent comprises unconverted ethane and ethylene, to the steam cracker configuration;
optionally recycling unconverted ethane from the stream comprising unconverted ethane coming from the C2 separation unit to the ODH unit.

2. Process according to claim 1, wherein the stream coming from the steam cracker unit of the steam cracker configuration comprises unconverted ethane, ethylene, hydrogen, methane, carbon monoxide, carbon dioxide and C3+ hydrocarbons; the stream coming from the ODH unit of the ODH configuration comprises unconverted ethane, ethylene, water, carbon monoxide and carbon dioxide; and the process comprises the following steps:
subjecting a stream comprising naphtha to steam cracking conditions in a steam cracker unit which is part of a steam cracker configuration, resulting in a stream comprising unconverted ethane, ethylene, hydrogen, methane, carbon monoxide, carbon dioxide and C3+ hydrocarbons;
feeding a stream comprising unconverted ethane, ethylene, hydrogen, methane, carbon monoxide, carbon dioxide and C3+ hydrocarbons to a carbon dioxide removal unit which is part of the steam cracker configuration, and removing carbon dioxide from said stream in said carbon dioxide removal unit resulting in a stream comprising unconverted ethane, ethylene, hydrogen, methane, carbon monoxide and C3+ hydrocarbons;
feeding a stream comprising unconverted ethane, ethylene, hydrogen, methane, carbon monoxide and C3+ hydrocarbons to a separation unit which is part of the steam cracker configuration, and separating said stream in said separation unit into a stream comprising hydrogen, methane and carbon monoxide and a stream comprising C3+ hydrocarbons;
feeding unconverted ethane and ethylene from a stream comprising unconverted ethane, ethylene, hydrogen, methane, carbon monoxide and C3+ hydrocarbons to a C2 separation unit which is part of the steam cracker configuration, and separating said stream in the C2 separation unit into a stream comprising ethylene and a stream comprising unconverted ethane;
optionally recycling unconverted ethane from the stream comprising unconverted ethane coming from the C2 separation unit to the steam cracker unit;
subjecting a stream comprising ethane to oxidative dehydrogenation (ODH) conditions in an ODH unit which is part of an ODH configuration, resulting in a stream comprising unconverted ethane, ethylene, water, carbon monoxide and carbon dioxide;
feeding a stream comprising unconverted ethane, ethylene, water, carbon monoxide and carbon dioxide to a water condensation unit which is part of the ODH configuration, and removing water from said stream by condensation in the water condensation unit, resulting in a stream comprising unconverted ethane, ethylene, carbon monoxide and carbon dioxide;
feeding an effluent coming from the ODH configuration, which effluent comprises unconverted ethane and ethylene, to the steam cracker configuration;
optionally recycling unconverted ethane from the stream comprising unconverted ethane coming from the C2 separation unit to the ODH unit.

3. Process according to claim 2, wherein:
1) in a 1^{st} embodiment, the stream comprising unconverted ethane, ethylene, hydrogen, methane, carbon monoxide and C3+ hydrocarbons is separated in the separation unit into a stream comprising hydrogen, methane and carbon monoxide and a stream comprising C2+ hydrocarbons, which C2+ hydrocarbons comprise unconverted ethane, ethylene and C3+ hydrocarbons;
the separated stream comprising C2+ hydrocarbons is fed to a further separation unit, wherein said stream is separated into a stream comprising C2 hydrocarbons, which C2 hydrocarbons comprise unconverted ethane and ethylene, and a stream comprising C3+ hydrocarbons; and
the separated stream comprising C2 hydrocarbons coming from the further separation unit is fed to the C2 separation unit wherein the latter stream is separated into a stream comprising ethylene and a stream comprising unconverted ethane; or
2) in a 2^{nd} embodiment, the stream comprising unconverted ethane, ethylene, hydrogen, methane, carbon monoxide and C3+ hydrocarbons is separated in the separation unit into a stream comprising hydrogen, methane, carbon monoxide and C2 hydrocarbons, which C2 hydrocarbons comprise unconverted ethane and ethylene, and a stream comprising C3+ hydrocarbons;
the separated stream comprising hydrogen, methane, carbon monoxide and C2 hydrocarbons is fed to a further separation unit, wherein said stream is separated into a stream comprising hydrogen, methane and carbon monoxide and a stream comprising C2 hydrocarbons; and
the separated stream comprising C2 hydrocarbons coming from the further separation unit is fed to the C2 separation unit wherein the latter stream is separated into a stream comprising ethylene and a stream comprising unconverted ethane.

4. Process according to any one of the preceding claims, wherein the stream coming from the steam cracker unit of the steam cracker configuration additionally comprises acetylene; the stream coming from the ODH unit of the ODH configuration additionally comprises acetylene; and the process comprises the following additional step:
an acetylene hydrogenation step wherein in an acetylene hydrogenation unit, which is part of the steam cracker configuration, a stream comprising unconverted ethane, ethylene and acetylene is subjected to hydrogenation conditions, so as to convert acetylene into ethylene.
